# EUROPEAN PATENT APPLICATION

(11) **EP 4 059 449 A1**
(43) Date of publication of application: **21.09.2022**
(21) Application number: 21382208.3
(22) Date of filing: 15.03.2021
(51) Int. Cl.: A61B 17/16, B23B 51/00

(54) **EXPANSIVE DRILL AND METHOD FOR DRILLING A HOLE USING SAID EXPANSIVE DRILL**

(71) Applicant: Fundación Tecnalia Research & Innovation, 20009 Donostia-San Sebastian, Guipuzcoa (ES)
(72) Inventor: ABASCAL MURO, José Miguel, 20009 Donostia-San Sebastian (Gipuzkoa) (ES)
(74) Representative: Balder IP Law, S.L.

(57) **Abstract**

The invention discloses an expansive drill (1) comprising: a body (2) configured for rotating around a longitudinal axis (A_{L}); at least one blade (31, 32) connected to a distal tip portion of the body (2) by means of a rotatable joint (4), where the rotatable joint (4) has an axis of rotation (A_{J}) perpendicular to the drill longitudinal axis (A_{L}); and a blade expansion mechanism causing the blade (31, 32) to selectively move between a retracted position and an extended position. The blade expansion mechanism is configured such that, by the blade (31, 32) moving gradually towards the extended position while the body (2) still rotates, the expansive drill (1) enlarges a distal end of the hole (H) for making the distal portion. The invention further discloses a method for making a hole (H) using the expansive drill (1) disclosed above.

## Description

### OBJECT OF THE INVENTION

A first object of the present invention is an expansive drill for making a hole of a first diameter ending in a cavity having a variable diameter and with a maximum diameter that is larger than the first diameter.

A second object of the present invention is a method for making a hole with the features disclosed above using said expansive drill.

### PRIOR ART

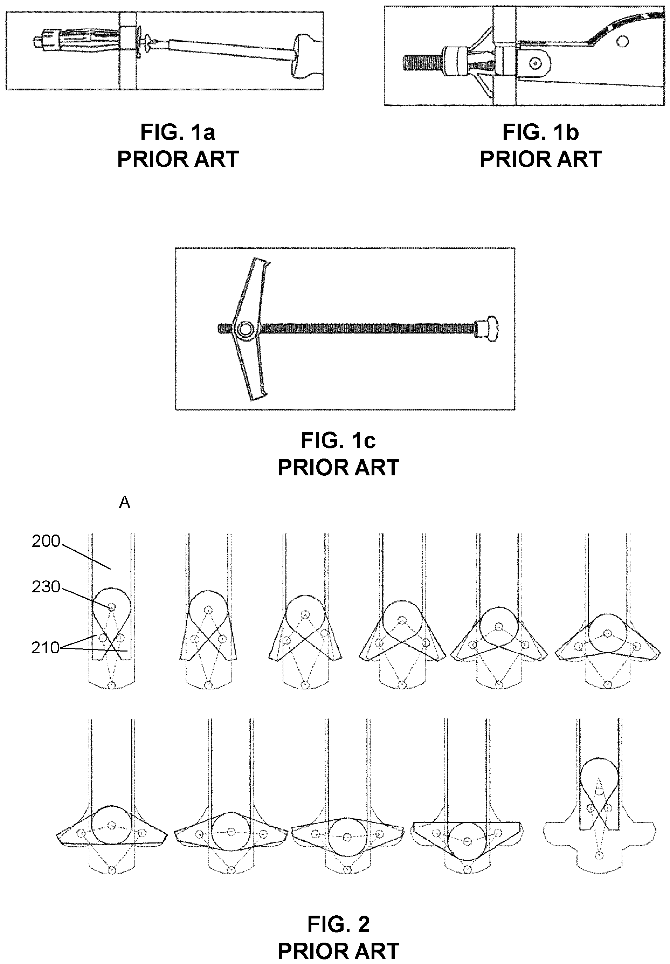

A great variety of folding dowels, like that shown in Figs 1a-c, can be found in the market. If this kind of folding dowels are used for providing a good fastening and fixation, an open surface behind one thin surface is needed. For this reason, they can't be used in large solid surfaces of any material.

But if a hole of a first diameter ending in a cavity having a maximum diameter larger than the first diameter could be made these kinds of folding dowels could be used in large solid surfaces. Some of the different folding dowels are adapted to different surface thickness, so the first diameter hole length should be adaptable. Also, the maximum diameter hole length should not be very long, because sometimes the surfaces are not large enough to contain a large length hole.

Document ES2301321 discloses an expansive drill for enlarging holes. This expansive drill comprises: a body (200) configured for rotating around the longitudinal axis (A) of the expansive drill; two blades (210) connected to a tip portion of the body (200) by means of a common rotatable joint (230), where the rotatable joint (230) has an axis of rotation oriented in perpendicular to the longitudinal axis (A) of the expansive drill; and a blade expansion mechanism coupled to the blades that causes the blades (210) to selectively move between a retracted position where the blades (210) do not protrude radially from the body (200) and an extended position where the blades (210) protrude radially from the body (200) essentially in perpendicular to the longitudinal axis (A). As shown in Fig. 2 (also Fig. 2 in document ES2301321), this expansive drill can enlarge a distal portion of a cylindrical hole made in advance by means of a different tool, e.g., by a conventional drill.

An important drawback of the expansive drill of document ES2301321 is that two different tools are required for making a hole having a proximal portion having a first diameter and a distal portion having a maximum diameter larger than the first diameter. Indeed, in order to make such a hole, first a conventional drill for making a cylindrical bore is employed, and then the expansive drill of document ES2301321 is used for enlarging a distal portion of the cylindrical bore. This is disadvantageous in that making a hole of this type takes a long time. Further, the need to employ two different tools is also more cumbersome for the operator.

Another disadvantage is that the expanding hole in the ES2301321 makes a cylindrical hole at a fixed distance and the different existing folding dowels are developed for surfaces of different thicknesses so, the cylindrical hole cannot normally be adapted to the space requirements of the different folding dowels.

One more drawback of the expansive drill of document ES2301321 is that a transition surface between the proximal portion and the distal portion of the hole is not contained in a plane perpendicular to the longitudinal axis of the hole. Thus, while the holes made with the expansive drill of document ES2301321 have a distal portion having a larger diameter, said holes are not useful for some folding dowels which need a perfect perpendicular plane to fix. This is because the blade expansion mechanism in document ES2301321, that is coupled to the blades, makes the expansive drill, and more particularly the blades, to move relative to the hole in the direction of the longitudinal axis during the operation for making the distal portion of the hole.

### SUMMARY

The expansive drill of the present invention solves the aforementioned drawbacks by means of a novel mechanism that causes the blades of the expansive drill to move between the retracted and extended positions. When in the retracted position, this mechanism allows a distal tip of each blade to be positioned in the longitudinal axis of the drill. The expansive drill can thus make a conventional cylindrical hole. Then, the expansive drill can cause each blade to move to their respective extended positions for enlarging the distal portion of the hole. Furthermore, a particularly preferred extended position of the blades can render a transition surface between the proximal portion and the distal portion of the hole that is essentially contained in a plane perpendicular to a longitudinal axis of the hole.

A number of terms and expressions used in the description of the present invention are disclosed herein below.

Proximal/distal: In the present document, these terms have the usual meaning they are given in the medical field. Namely, the term "*proximal*" refers to a side, end, or portion located closest to the user of the expansive drill of the invention, while the term *"distal"* refers to a side, end or portion located farthest to the user of the expansive drill of the invention.

Longitudinal axis: In the present document, two longitudinal axes are disclosed. The cylindrical hole has a first longitudinal axis, and the expansive drill has a second longitudinal axis. When the expansive drill is in use, i.e., drilling a hole, the two longitudinal axes are coincident. Generally speaking, unless the context indicates otherwise, references in the present document to a *"longitudinal axis"* refer to the longitudinal axis of the drill.

### Expansive drill

A first aspect of the present invention is directed to an expansive drill for drilling a hole. The expansive drill comprises: a body configured for rotating around a longitudinal axis of the drill; at least one blade connected to a distal tip portion of the body by means of a rotatable joint, where the rotatable joint has an axis of rotation oriented in perpendicular to the drill longitudinal axis; and a blade expansion mechanism configured to cause the blade to selectively move between a retracted position where the blade does not protrude radially from the body and an extended position where the blade protrudes radially from the body. In a particularly preferred embodiment of the invention, in the extended position the blade protrudes radially from the body essentially in perpendicular to the longitudinal axis of the drill, thereby rendering a hole having a transition surface between the proximal portion and the distal portion that is essentially contained in a plane perpendicular to the longitudinal axis of the hole.

The above features making up the preamble of the independent claim correspond to features known from document ES2301321. The expansive drill of the present invention differs from said prior art drill in that the blade expansion mechanism is located in the body and in that the expansive drill is configured such that, with the blade in the retracted position, a pointed distal tip of the blade is positioned in correspondence with the longitudinal axis of the drill. Therefore, when the body rotates with the blade in the retracted position, the expansive drill makes a proximal portion of the hole and, by actuation of the blade expansion mechanism, the blade moves gradually towards the extended position while the body still rotates, the blade of the expansive drill enlarges a distal end of the hole for making a distal portion of the hole. Thus, the hole will have a proximal portion having a first diameter and a distal portion having variable diameter with a maximum diameter larger than the first diameter. Due to the fact that the blade expansion mechanism is located within the body, the expansion drill does not have to move relative to the hole during the operation of making the distal portion of the hole. So, the shape and dimensions of the cavity created is determined by the shape and dimensions of the blades and their movement relative to the body when they are extended.

Since the blade is usually a flat blade, the pointed distal tip is formed by two edges of the blade meeting in a variable angle, e.g., an angle of 75° or less. When such a pointed distal tip is provided in the axis of rotation of the drill, i.e., the longitudinal axis of the drill, the drill is naturally well suited for starting a hole. The user can then use the expansive drill of the invention as a conventional drill for boring a cylindrical hole. Once a distal portion of the body is introduced sufficiently far into the part where the hole is bored, the user can cause the blade to move to the extended position for enlarging the distal portion of the hole. As mentioned above, this configuration is advantageous in that only one tool is required for making the hole, either the conventional cylindrical hole or the hole with the enlarged cavity located at its distal end.

In principle, the blade expansion mechanism could be any mechanism and may be configured in any way provided that the user can easily actuate such mechanism for selectively moving the blade from its retraced position to its extended position in order to bore a conventional cylindrical hole or enlarging a distal portion of said cylindrical hole. In particular, according to a preferred embodiment of the invention, the blade expansion mechanism comprises a longitudinal rod housed within the body, where the longitudinal rod is longitudinally displaceable with respect to said body between a proximal position and a distal position. Further, the longitudinal rod is connected to the blade such that movement of the longitudinal rod between the proximal position and the distal position causes the blade to move between the retracted position and the extended position. Even more preferably, the connection between the longitudinal rod and the blade comprises a distal longitudinal rack of the longitudinal rod threaded to a proximal pinion of the blade. The proximal pinion may form an integral part with the blade or may be coupled to the blade by welding, adhesive or any other mechanical means.

This configuration is advantageous because a simple and convenient manner to move the blade between the retracted and extended positions is provided. Further, a longitudinally sliding rod having a rack engaged to a pinion of the blade provides an essentially unlimited range of rotation for the blades.

In a further preferred embodiment of the present invention, the expansive drill further comprises a spring that longitudinally extends between the body and the longitudinal rod. The spring connects the body with the longitudinal rod and is configured to continuously bias the longitudinal rod towards the proximal or distal position. Even more preferably, the expansive drill further comprises a latch mechanism for latching the longitudinal rod in a desired position. Since different spring and rod configurations could be provided, in principle the desired position could be any one of the proximal or distal position. Thus, when the user is not holding the rod, the rod naturally moves to a particular rest position. This rest position would normally be the position corresponding to the retracted position of the blade. Therefore, the user could use the expansive drill of the invention for boring a conventional cylindrical hole and, and once it has been made, the user could move the rod longitudinally against the force provided by the spring in order to reach the position of the rod corresponding to the extended position of the blades. The user could then latch the rod in said position until the distal portion of the hole is enlarged as desired.

This configuration is advantageous in that the blades are ensured to be in a particular position at all times unless the user actively causes them to move to a different position.

In a particularly preferred embodiment of the invention, the body further comprises an alignment radial pin housed within a longitudinal groove of the rod for guiding the longitudinal displacement of the rod.

This configuration is advantageous in that the provision of a radial pin ensures a correct longitudinal displacement of the rod with respect to the body.

In still one more preferred embodiment of the present invention, the blade has an essentially trapezoid shape, e.g., a rectangular, isosceles, acute, etc., trapezoid shape, where the rotatable joint is provided at a vertex of the trapezoid, and where a cutting edge of the blade is, at least, provided at a side of the trapezoid opposite said vertex. Rectangular trapezoid shaped blades are advantageous in that the pointed tip of the expansive drill formed by the blades, in its retracted position, has the geometry of conventional drill bits, said geometry being more efficient for drilling holes than other geometries. Alternatively, the blade may have a substantially triangular shape where the rotatable joint is provided at a vertex of the triangle, and where a cutting edge of the blade is, at least, provided at a side of the triangle opposite said vertex. More particularly, the essentially triangular shape is an essentially right triangle shape where the rotatable joint is provided at the right vertex of the triangular blade. Triangular blades are advantageous in that a pointed tip as disclosed above is naturally provided. The blades may have any other shape provided that they are able to bore the cylindrical hole in their retracted position and the enlarged cavity in their extended position.

In one more preferred embodiment of the invention, the expansive drill comprises two blades connected to the distal tip end of the body by means of a common joint. The two blades are configured for synchronously moving between the retracted position and the extended position. Then, when in the retracted position, both pointed tips of the two blades are positioned at the same position in the longitudinal axis of the drill, such that they both combine for rendering a drilling tip suitable for boring a hole.

This configuration is advantageous because the resulting expansive drill has a symmetrical configuration where a correct distribution of the forces appearing during use is easier. Furthermore, using two blades instead of one allows for lower hardness requirements for each of the blades.

According to one more preferred embodiment of the invention, the expansive drill comprises stop means, for example, an annular portion protruding radially from the body for limiting a depth of the hole made by the expansive drill. The stop means could in principle have any configuration provided they bump against a surface of the part being drilled once the hole has a particular depth. For example, the stop means could be a ring part coupled to the body. Said ring part would protrude radially from said body out of the hole made by the blade. The position of the stop means relative to the distal tip of the expansible drill can be adjusted. By adjusting the position of the stop means relative to the distal tip of the expansible drill, the depth of the cylindrical hole bored by the expansive drill, and thus, the depth at which the enlarged cavity is made, can be adjusted by the user. For example, the body of the expansive drill may comprise guides through which the stop means may slide to adjust its position relative to the distal tip of the expansive drill. The guides may integrate a retaining mechanism for retaining the stop means in the desired position relative to the body. The body and the stop means may integrate any other mechanism to allow the movement of the stop means relative to the body. In some other embodiments, the position of the stop means relative to the distal tip of the expansive may be fix.

The provision of stop means is advantageous in that a hole where the transition surface between the proximal portion and the distal portion is perpendicular to the longitudinal axis of the hole is easier to obtain. Indeed, the user needs only to first bore the proximal portion of the hole with the blades in the retracted position and, only when the stop means abuts against the surface of the part being drilled, to then cause the blades to move from the retracted position to the extended position. The adjustable stop means also allows to adjust the depth of the hole to be made in the part.

### Method for drilling a hole using the expansive drill

A second aspect of the present invention is directed to a method for drilling a hole. The method comprises mainly the following steps:
1. Positioning the distal tip of the blade of an expansive drill as disclosed above, with the blade in the retracted position, against a surface of a part where the hole is going to be drilled;
2. Rotating the body of the expansive drill while pushing distally along the longitudinal axis of the drill for causing the blade to make the proximal portion of the hole as a distal portion of the body is introduced into the part, the proximal portion of the hole (H) having a first diameter (D₁);
3. Causing, by the blade expansion mechanism, the blade to move gradually from the retracted position to the extended position while maintaining the body continuously rotating, for causing the blade to make a distal portion of the hole, the distal portion having a maximum diameter (D₂) larger than the first diameter (D₁); and
4. Causing, by the blade expansion mechanism, the blade to move back to the retracted position for extracting the distal portion of the body from within the hole.

In a preferred embodiment of the method of the invention, the method comprises abutting the stop means against the surface of the part and while the stop means abut against said surface causing the blade to move gradually from the retracted position to the extended position to make the distal portion of the hole.

In another preferred embodiment of the method of the invention, the step of causing the blade move between the retracted position and the extended position comprises moving the longitudinal rod between a proximal position and a distal position.

In a more preferred embodiment of the method of the invention, the step of moving the longitudinal rod between a proximal position and a distal position comprises latching the longitudinal rod in a desired position.

In another preferred embodiment of the method of the invention, prior to positioning the distal tip of the blade against the surface of the part where the hole is going to be drilled, the position of the stop means relative to the distal tip of the blade is adjusted to determine the depth of the hole to be drilled.

### BRIEF DESCRIPTION OF THE DRAWINGS

Figs. 1a-1c show several examples of folding dowels.
Fig. 2 schematically shows the operation of an embodiment of an expansive drill according to the prior art.
Figs. 3a-3d show the operation of an example of an expansive drill according to the present invention.
Fig. 4 shows a perspective view of an example of an expansive drill according to the present invention.
Figs 5a-5c show the operation of an example of blade expansion mechanism according to the present invention.
Fig. 6 shows a first exemplary embodiment showing a movement of the blades of the expansive drill of the present invention.
Fig. 7 shows a second exemplary embodiment showing an alternative movement of the blades of the expansive drill of the present invention.
Figs. 8a and 8b show two different holes made by the expansive drills of Figures 6 and 7, respectively.
Figs. 9a-c show the operation of an example of an expansive drill according to the present invention, with the stop means located in different positions.

### DESCRIPTION OF A PREFERRED EMBODIMENT

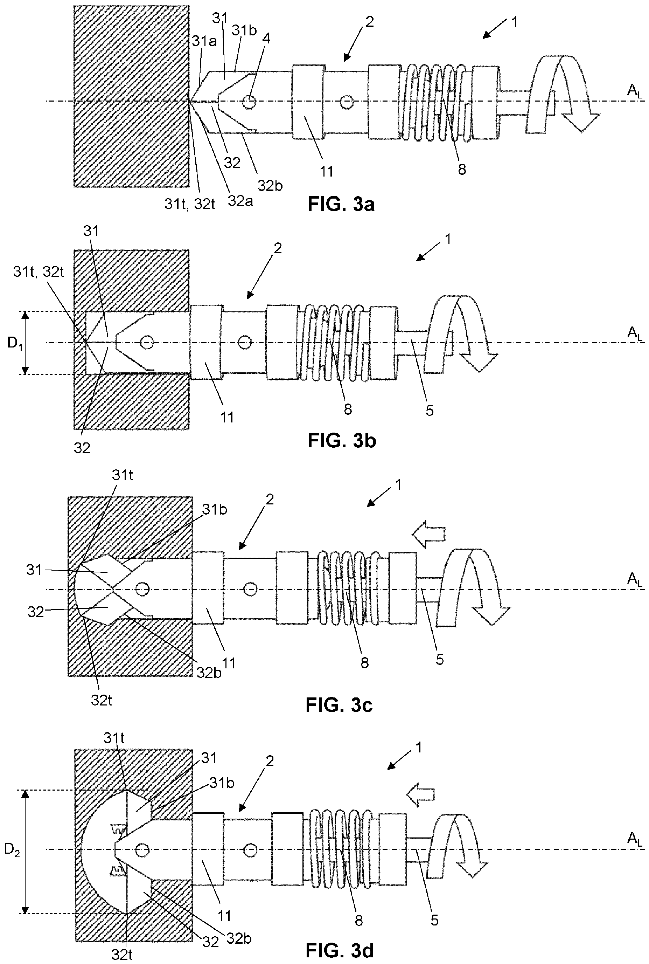

Figs. 3a-3d show an expansive drill (1) according to the present invention in different steps of a process for making a hole (H) comprising a proximal portion having a first diameter (D₁) and a distal portion having a maximum diameter (D₂) larger than the first diameter (D₁). In this particular example, the extended position of the blades (31, 32) is selected for rendering a transition surface between the proximal portion and the distal portion of the hole (H) essentially contained in a plane that is perpendicular to a longitudinal axis of the hole (H).

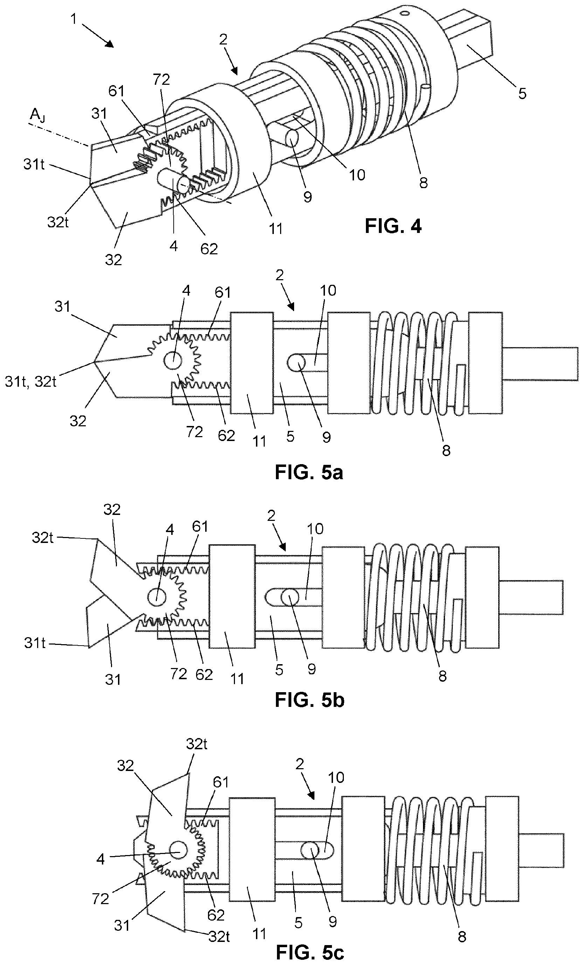

The expansive drill (1) of the invention comprises a rotatable body (2) around a longitudinal axis (A_{L}) of the expansive drill (1). Thereto, the body (2) includes a longitudinal rod (5) whose free end is to be connected to a conventional drill or to a rotary motor (not shown in the figures). This free end of the rod (5) is normally inserted into a receiving cavity of the drill or motor which is connected by interposition of a rotary axis to an electric rotary motor. The body (2) is essentially cylindrical having a distal portion and a proximal portion. At the tip of the distal portion, two essentially planar trapezoid blades (31, 32) are provided. As shown in more detail in Figs. 4-6, the blades (31, 32) have an essentially rectangular trapezoid shape where the joint (4) is located at the right angle of the rectangular trapezoid blade. The two blades (31, 32) are connected to a distal tip of the body (2) by means of a single common rotatable joint (4) having an axis of rotation (A_{J}) contained in a plane perpendicular to the longitudinal axis (A_{L}). The blades (31, 32) are rotatable between a retracted position and an extended position.

In the retracted position, as shown in Figs. 3a and 3b, the two blades (31, 32) are positioned such that a pointed distal tip (31t, 32t), i.e., a distal vertex, of both blades (31, 32) is positioned in the longitudinal axis (A_{L}) of the drill (1). In this retracted position, an essentially longitudinally aligned edge of the two blades (31, 32) is slightly juxtaposed, such that both blades (31, 32) combine for rendering an essentially single pointed tip oriented in the longitudinal direction (A_{L}). The pointed tip of the expansible drill (1) has essentially the same shape than a conventional drill. One of the outer edges (31a,32a) of the two blades (31, 32) having a cutting edge is oriented diagonally outwards with respect to the longitudinal axis (A_{L}) while, at the same time, the other outer edge (31b,32b) of the two blades (31, 32) is aligned with the outer perimeter of the body (2). Thus, in their retracted position, no part of the blades (31, 32) protrudes radially from the body (2). In such a retracted position, the two blades (31, 32) make up a cutting tip for the expansive drill (1) of the present invention capable of boring a conventionally cylindrical hole having the first diameter (D₁). The first diameter (D₁) is essentially coincident with the diameter of the body (2). Thus, in the step shown in Fig. 3a, the cutting tip conformed by the respective pointed tips (31t, 32t) of the blades (31, 32) of the expansive drill (1) of the present invention abuts against the surface of a part to be drilled. Then, as shown in Fig. 3b, the user pushes longitudinally the expansive drill (1) against the part to be drilled. The distal portion of the body (2) enters into the part as the blades (31, 32) bore a cylindrical hole (H) having the first diameter (D₁).

Then, the blades (31, 32) are moved towards the extended position. In the extended position, each blade has rotated around the rotatable joint (4) until a position where the outer edge (31b,32b) having the cutting edge is perpendicular to the longitudinal axis (A_{L}). The pointed tip (31t, 32t) of each blade (31, 32) thus leaves the original position on the longitudinal axis (A_{L}) and moves to opposite positions that protrude radially from the body (2) of the drill (1). The body (1), and therefore also the blades (31, 32), still rotates around the longitudinal axis (A_{L}). Consequently, the distal portion of the hole (H) is enlarged gradually as the blades (31, 32) rotate around the joint (4), see Fig. 3c. At the end of this step, the blades (31, 32) have arrived at the extended position where the cutting edge protrudes radially outwards from the body (2) in perpendicular to the longitudinal axis (A_{L}). In this situation, as shown in Fig. 3d, a distal portion of the hole (H) is created having a maximum diameter (D₂) that is naturally larger than the first diameter (D₁) of the proximal portion of the hole (H). Since the cutting edge of the blades (31, 32) in the extended position is perpendicular to the longitudinal axis (A_{L}), a transition surface between the proximal portion and the distal portion of the hole (H) is also contained in a plane that is perpendicular to said longitudinal axis (A_{L}).

Further, note that the expansive drill (1) of the present invention further comprises stop means (11) connected to the body (2). The stop means are an annular piece that protrudes radially from the body (2) such that, as the expansive drill (1) moves into the part to be drilled, there is a moment where the stop means (11) abut against the surface of the part surrounding the hole (H). The body (2) of the expansive drill (1) can no longer move into said part, i.e., the progression of the expansive drill (1) inside the part in the direction of the longitudinal axis (A_{L}) is blocked. It is at this moment when, by keeping pushing longitudinally the expansive drill (1) against the part to be drilled, the blades (31, 32) are caused to move from the retracted position to the extended position. Since the progression of the expansive drill (1) is thus blocked, the body (2) does not move relative to the hole (H) during the operation of making the distal portion of the hole (H) so that the transition surface between the distal portion and the proximal portion of the hole (H) carved by the blades (31, 32) is contained in a plane perpendicular to the longitudinal axis (A_{L}).

Figs. 4-5 show in more detail the blade expansion mechanism causing the blades (31, 32) to move between the retracted and expanded positions. A longitudinal rod (5) is provided within the body (2). In such embodiment, the blades (31, 32) move relative to each other in an opposite direction than the blades shown in the expansive drill of Figure 3. The longitudinal rod (5) is longitudinally displaceable along the body (2) between a proximal position and a distal position. The longitudinal rod (5) further comprises an enlarged longitudinal groove (10) along which an alignment radial pin (9) of the body (2) slides for ensuring a correct longitudinal movement of the rod (5) with respect to the body (2). A spring (8) connected between the longitudinal rod (5) and the body (2) continuously biases the longitudinal rod (5) towards the proximal position. If the user keeps pushing longitudinally the expansive drill (1) against the part to be drilled once the stop means abut against the surface of the part, the spring (8) is compressed allowing the longitudinal rod (5) move between its proximal position and its distal position causing the blades (31, 32) to move from the retracted position to the extended position.

A distal portion of the longitudinal rod (5) further comprises two toothed distal longitudinal racks (61, 62). A first longitudinal rack (61) of the longitudinal rod (5) is engaged to a toothed proximal pinion (not visible in the figures) of a first blade (31), while a second longitudinal rack (62) of the longitudinal rod (5) is engaged to a toothed proximal pinion (72) of a second blade (32). When the longitudinal rod (5) moves longitudinally, the interaction between the racks (61, 62) and the pinions (72) causes the blades (31, 32) to rotate around the common joint (4). Further, note that each longitudinal rack (61, 62) is engaged to opposite sides of the respective pinions (72), such that a longitudinal displacement of the racks (61, 62) would cause the respective pinions (72) to rotate in opposite directions. Thus, by pushing or pulling longitudinally rod (5), the blades (31, 32) are caused to move between the retracted and extended positions.

Fig. 5a shows the longitudinal rod (5) in a proximal position. In this situation, the two blades (31, 32) are positioned such that their pointed tips (31t, 32t) are situated in correspondence with the longitudinal axis (A_{L}). The longitudinal rod (5) is then pushed distally against the force of the spring (8) in the longitudinal direction. Racks (61, 62) cause the pinions (72) to rotate in opposite directions, and therefore each blade (31, 32) rotates such that the pointed tips (31t, 32t) thereof move outwards further than a diameter of the body (2). In this situation, shown in Fig. 5b, the enlarged distal portion of the hole (H) starts to be made (see also Fig. 3c). Finally, when the longitudinal rod (5) reaches the distal position, the two blades (31, 32) adopt the extended position where the cutting edges of the blades are perpendicular to the longitudinal axis (A_{L}). In this situation, shown in Fig. 5c, the transition plane between the proximal portion and the distal portion of the hole (H) is carved.

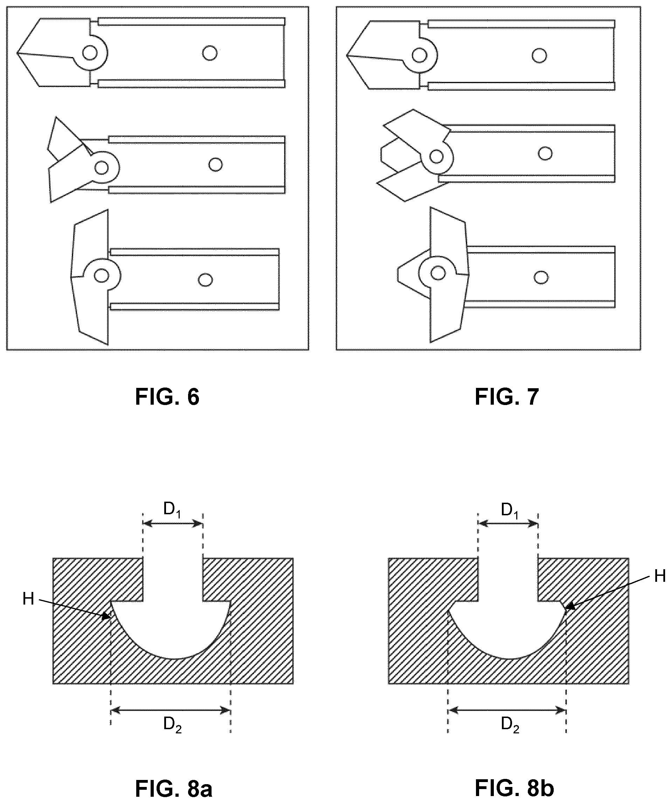

Figs. 6 and 7 show two configurations of the movement of the blades (31, 32) caused by the blade expansion mechanism. In Fig. 6, the blades (31, 32) rotate around the joint (4) in respective first directions, such that the cutting tip of each blade (31, 32), when in the extended position, is oriented diagonally outwards with respect to the longitudinal axis (A_{L}). In Fig. 7, the blades (31, 32) rotate around the joint (4) in respective second directions opposite to the first directions, such that the cutting tip of each blade (31, 32), when in the extended position, is oriented perpendicularly outwards with respect to the longitudinal axis (A_{L}).

Figs. 8a and 8b show the resulting hole (H) made by means of the expansive drills (1) shown in figures 6 and 7, respectively. In both cases, the hole (H) has a proximal cylindrical portion having a first diameter (D₁) and an enlarged distal portion having a maximum diameter (D₂) larger than the first diameter (D₁). In Figure 8a, since the blades (31,32) rotate around the joint (4) in the first direction, the enlarged distal portion of the hole has its maximum diameter (D₂) adjacent the proximal portion of said hole (H). Thus, there is one single transition surface between the proximal and distal portions of the hole (H) that is contained in a plane that is perpendicular to the longitudinal axis of the hole (H). In figure 8b, since the blades (31,32) rotate around the joint (4) in the second direction, the enlarged distal portion of the hole has its maximum diameter (D₂) in a plane that is close the proximal portion of said hole (H). In such case, there are two transition surfaces between the proximal and distal portions of the hole (H). A first transition surfaces that is contained in a plane that is perpendicular to the longitudinal axis of the hole (H) and a second transition surface that is inclined relative to the first transition surface and whose inclination corresponds to the inclination of the outer edges (31a,32a) of the two blades (31, 32) relative to the longitudinal axis (A_{L}), when fully expanded.

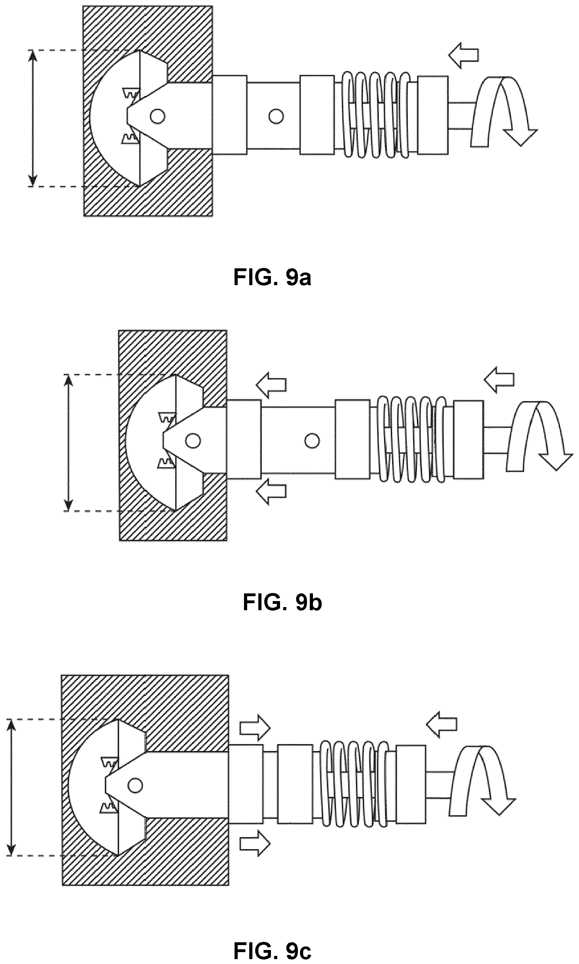

Figs. 9a-c show the operation of an example of an expansive drill (1) according to the present invention, with the adjustable stop means (11) located in different positions. In figure 9a the adjustable stop means (11) have been previously fixed in a position that substantially corresponds with the center of the section of the body (2) located between the spring (8) and the blades (31,32). In figure 9b the adjustable stop means (11) have been previously fixed in a position that is close to the blades (31,32) to drill a not very deep hole (H). In figure 9c the adjustable stop means (11) have been previously fixed in a position that is close to the spring (8) to drill a deeper hole (H). By adjusting the position of the stop means (11) relative to the distal tip portion of the blades (31,32), holes of different depths can be bored in the part. The distance between the distal tip portion of the blades (31,32) and the abutting surface of the stop means (11) will determined the depth of the hole (H).

## Claims

1. Expansive drill (1) for drilling a hole (H), the expansive drill (1) comprising:
- a body (2) configured for rotating around a longitudinal axis (A_{L}) of the expansive drill (1);
- at least one blade (31, 32) connected to a distal tip portion of the body (2) by means of a rotatable joint (4), where the rotatable joint (4) has an axis of rotation (A_{J}) oriented in perpendicular to the drill longitudinal axis (A_{L}); and
- a blade expansion mechanism that is configured to cause the blade (31, 32) to selectively move between a retracted position where the blade (31, 32) does not protrude radially from the body (2) and an extended position where the blade (31, 32) protrudes radially from the body (2),
**characterized in that** the blade expansion mechanism is located in the body (2) and the expansive drill (1) is configured such that, with the blade (31, 32) in the retracted position, a pointed distal tip (31t, 32t) of the blade (31, 32) is positioned in correspondence with the drill longitudinal axis (A_{L}), such that, when the body (2) rotates with the blade (31, 32) the expansive drill (1) makes a proximal portion of the hole (H) and, by actuation of the blade expansion mechanism, the blade (31, 32) moves gradually towards the extended position while the body (2) still rotates, the blade enlarging a distal end of the hole (H) for making a distal portion of the hole (H).

2. Expansive drill (1) according to claim 1, where the blade expansion mechanism comprises a longitudinal rod (5) housed within the body (2), the longitudinal rod (5) being longitudinally displaceable with respect to said body (2) between a proximal position and a distal position, where the longitudinal rod (5) is connected to the blade (31, 32) such that movement of the longitudinal rod (5) between the proximal position and the distal position causes the blade (31, 32) to move between the retracted position and the extended position.

3. Expansive drill (1) according to claim 2, where the connection between the longitudinal rod (5) and the blade (31, 32) comprises a distal longitudinal rack (61, 62) of the longitudinal rod (5) threaded to a proximal pinion (72) of the blade (31, 32).

4. Expansive drill (1) according to any of claims 2-3, further comprising a spring (8) that extends longitudinally between the body (2) and the longitudinal rod (5), the spring (8) being configured to continuously biasing the longitudinal rod (5) towards the proximal or distal position.

5. Expansive drill (1) according to claim 4, further comprising a latch mechanism for latching the longitudinal rod (5) in a desired position.

6. Expansive drill (1) according to any one of claims 2-5, where the body (2) further comprises an alignment radial pin (9) housed within a longitudinal groove (10) of the rod (5) for guiding the longitudinal displacement of the rod (5).

7. Expansive drill (1) according to any one of the previous claims, wherein the blade (31, 32) has an essentially trapezoid shape, where the rotatable joint (4) is provided at a vertex of the trapezoid, and where at least one of the cutting edges of the blade (31, 32) is provided at a side of the trapezoid opposite to said vertex.

8. Expansive drill (1) according to claim 7, where the essentially trapezoid shape is an essentially rectangular trapezoid shape where the rotatable joint (4) is provided at the right vertex of the trapezoid shaped blade (31, 32).

9. Expansive drill (1) according to any one of the previous claims, comprising two blades (31, 32) connected to the distal tip portion of the body (2) by means of a common joint (4), the two blades (31, 32) being configured for synchronously moving between the retracted position and the extended position.

10. Expansive drill (1) according to any one of the previous claims, further comprising stop means (11) protruding radially from the body (2) for limiting a depth of the hole (H) made by the expansive drill (1).

11. Expansive drill (1) according to claim 10, comprising means for adjusting the position of the stop means (11) relative to the distal tip portion of the blades (31,32).

12. Method for drilling a hole (H) using the expansive drill (1) according to any one of the preceding claims, the method comprising the steps of:
- positioning the distal tip (31t, 32t) of the blade (31, 32) of the expansive drill (1), with the blade (31, 32) in the retracted position, against a surface of a part where the hole (H) is going to be drilled;
- rotating the body (2) of the expansive drill (1) while pushing distally along the longitudinal axis (A_{L}) of the drill (1) for causing the blade (31, 32) to make a proximal portion of the hole (H) as a distal portion of the body (2) is introduced into the part, the proximal portion of the hole (H) having a first diameter (D₁);
- causing, by the blade expansion mechanism, the blade (31, 32) to move gradually from the retracted position to the extended position while maintaining the body (2) continuously rotating, for causing the blade (31, 32) to make a distal portion of the hole (H), the distal portion having a maximum diameter (D₂) larger than the first diameter (D₁); and
- causing, by the blade expansion mechanism, the blade (31, 32) to move back to the retracted position for extracting the body (2) from within the hole (H).

13. Method for drilling a hole (H) according to claim 12, comprising abutting the stop means (11) against the surface of the part and, while the stop means abut against the surface of the part, causing the blade (31, 32) to move gradually from the retracted position to the extended position to make the distal portion of the hole (H).

14. Method for drilling a hole (H) according to claim 12 or 13, where the step of causing the blade (31, 32) to move between the retracted position and the extended position comprises moving the longitudinal rod (5) between a proximal position and a distal position.

15. Method for drilling a hole (H) according to claim 14, where the step of moving the longitudinal rod (5) between a proximal position and a distal position comprises latching the longitudinal rod (5) in a desired position.
